(19) Europäisches Patentamt
European Patent Office
Office européen des brevets

(11) Publication number: **0 125 808 A2**

(12) **EUROPEAN PATENT APPLICATION**

(21) Application number: **84302641.0**

(22) Date of filing: **18.04.84**

(51) Int. Cl.³: **A 61 B 3/10**

(30) Priority: **18.04.83 US 486031**

(43) Date of publication of application: **21.11.84 Bulletin 84/47**

(84) Designated Contracting States: **DE FR GB IT NL**

(71) Applicant: **Weinblatt, Lee S.**
**797 Winthrop Road**
**Teaneck New Jersey 07666(US)**

(72) Inventor: **Weinblatt, Lee S.**
**797 Winthrop Road**
**Teaneck New Jersey 07666(US)**

(74) Representative: **Beresford, Keith Denis Lewis et al,**
**R.G.C. Jenkins & Co. 12-15, Fetter Lane**
**London EC4A 1PL(GB)**

(54) **Eye movement monitoring technique.**

(57) An eye movement detection scheme is provided whereby both a subject's eye movements and the scene being viewed can be monitored simultaneously with compact, light, unobtrusive yet effective equipment. Scene-mounting as well as eye-movement-monitoring devices (19, 13) are mounted on a spectacle frame (1) worn by the subject. Signals from these devices are conveyed electrically and/or optically from these frame-mounted devices to circuitry (23) for generating video compatible signals so that the eye movements can be superimposed over the scene for purposes of observation and analysis. An additional advantage is a feature which facilitates use of corrective lenses suitable to the individual subject so that eye-glass wearers can use the equipment as well.

FIG. 1

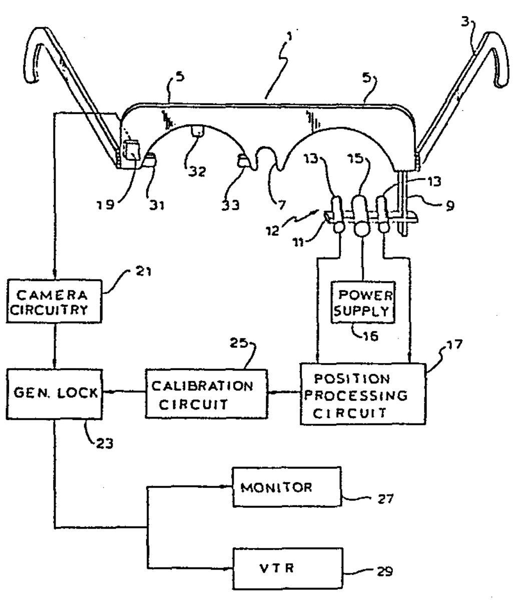


EP 0 125 808 A2

Croydon Printing Company Ltd.

# EYE MOVEMENT MONITORING TECHNIQUE

## Background of the Invention

The present invention relates to a technique for superimposing the detected eye movements of a viewer on the scene being viewed to display what in the scene is being looked at in any given moment, and in particular to a highly portable, head-mounted apparatus for simultaneously monitoring eye movements and recording the scene being viewed.

A need exists for an apparatus that can monitor the eye movements of a person in response to certain visual stimuli. Typically, the person would be exposed to a visual stimulus and his ocular reactions recorded by a monitoring apparatus. Such an apparatus can include a light source, visible or infrared, which is bounced off the eye into a suitable detector. The detected signal is then electronically processed to obtain a reading of the eye position at any given time.

Many applications exist for such an apparatus. These include medical diagnosis, military uses such as weapons aiming, training equipment such as aircraft simulators, sports analysis for improving visual techniques and concentration, advertisement testing, design planning as for an automobile dashboard, and testing for visual impact as of highway and store signs. In some of the listed applications, as for medical diagnosis and aircraft simulators, the eye-movement-monitoring apparatus is stationary, as is the equipment for presenting the visual stimuli, such as a television monitor. Since the latter two are fixed, the viewer can also be stationary. Typically, the subject is seated and his head fixed in place by a chin rest or a bite plate. However, in some applications, the exposure to the requisite stimuli requires movement. Thus, if analysis of a baseball batter's vision as he watches a pitched ball is desired, it would be preferable to actually do that in a batter's box in a realistic situation. Likewise, in advertising applications a subject may be requested to walk down a supermarket aisle so that his response to the most eye-catching containers can be recorded. Stationary equipment obviously cannot accomplish such tasks.

Head-mounted eye-movement-monitoring equipment has been devised which obviates the need to keep the person's head fixed. Since the equipment is affixed to the subject's head, it moves with his head and provides an accurate signal regardless of how he moves it. Such devices have been used in, for example, military applications where head movement is essential and even in applications where head movement is not essential but preferable. As regards the latter, using a fixed position for the head is to be avoided if the monitoring session is relatively lengthy because the subject is likely to experience considerable discomfort and a commensurate decrease in concentration. A head-mounted system in such a case is preferred. Various head-mounted arrangements are available. For example, U.S. Patent No. 4,102,565 issued July 25, 1978 to Henry L. Michael discloses a type of arrangement including light sources and sensors mounted on a helmet. U.S. Patent No. 3,473,868 issued October 21, 1969 to Young et al discloses a spectacle frame used in eye glasses. On the frame are mounted a light source and detectors to sense the light reflected by the eye to provide horizontal and vertical eye movement measurements.

For specific applications, regardless of whether stationary or head-mounted equipment is used, it is desirable to superimpose the subject's eye movements on the viewed scene. This, of course, requires not only eye movement monitoring equipment but also apparatus for recording the scene being viewed. Signals from the eye-movement-monitoring equipment and the scene recording equipment are electronically combined to attain the desired superimposition. Thus, in the above-described sports situation, the batter's eye movements would be superimposed on a scene showing approach of the pitched ball toward him. Analysis is thus possible of whether he is looking properly at the ball. In advertising, a subject is shown a filmed advertisement and his response monitored. By determining the portions of the advertisement to which the subject's eyes are drawn, an analysis is possible of the effectiveness of the advertisement in attracting the subject's attention and how it might be improved. See for example my U.S. Patent No. 4,075,657 issued February 21, 1978.

The present invention involves two of the features discussed above, namely portability and superimposition. It is desirable to provide a highly portable eye-movement-monitoring apparatus while adding to it a scene recording device. The two aims seem contradictory because one requires added weight and bulk while the other requires a high degree of portability, but the goal is achieved with the invention.

Equipment exists for accomplishing both aims of portability and recordal of the viewed scene. See, for example, U.S. Patent No. 3,542,457 issued November 24, 1970 to Balding et al. It discloses two helmet-mounted television cameras. One records an eye spot generated by an optical system for monitoring eye movement. The other is aimed at the scene being viewed. Signals from the two cameras are mixed to obtain the requisite superimposition. However, this and systems like it suffer from several disadvantages. The bulk and weight of the equipment can cause fatigue. Also, the equipment blocks the vision of one eye and reduces peripheral vision in the other. This interferes with depth perception and is a constant reminder of the test situation and can, therefore, result in distorted responses. Eye-glass wearers cannot use the equipment because the lenses can distort the light from the optical monitoring equipment and since the apparatus leaves too little clearance to accommodate glasses. In addition, such equipment cannot readily be calibrated for the eye shape of individual subjects.

## Summary of the Invention

It is, therefore, a general object of the present invention to provide a new and improved eye movement monitoring apparatus.

A more specific object of the present invention is to provide a new and improved eye movement monitoring apparatus combined with scene recordal equipment to enable superimposition of the subject's eye movements over the scene being viewed.

Another object of the present invention is to provide light, compact, highly portable and minimally intrusive eye movement monitoring and scene recordal equipment.

A further object of the present invention is to provide head-mounted eye movement monitoring and scene recordal equipment which can be used on subjects who wear eye glasses.

A still further object of the present invention is to provide superimposition of eye movements on the scene being viewed which provides ready calibration to individual eye shapes of subjects.

To accomplish these and other objects of the invention, an apparatus is provided which utilizes a spectacle frame as a support on which the scene monitoring and eye-movement monitoring devices are mounted. In various embodiments of the invention, such devices can be electronic and/or optical. They must be compact, light and minimally intrusive. Thus, in one embodiment a CCD camera is used to monitor the scene while a light source and detectors monitor the eye movements. In another embodiment, a CCD camera is used to monitor the scene, and another CCD camera is used to monitor eye movements. Further embodiments use optical lenses to monitor both the scene and eye movements. Light from the lenses is combined by feeding light from each lens into a separate video camera and then combining the signals electronically, or by first combining the light optically, as with a semi-silvered mirror, and then recording the result with a video camera.

In all of the above embodiments, the apparatus worn by the subject is minimized as to size and weight. The signals picked up by the scene and eye-movement detectors are picked up by the light and compact spectacle-frame-mounted devices and conveyed elsewhere for processing. The processing circuitry can, for example, be on the person of the subject or in another location.

To enable eye glass wearers to use this equipment, the spectacle frame is adapted for convenient insertion and removal of corrective lenses. Corrective lenses suitable to the individual can be selected from a stock kept on hand and then placed in the frame.

## Brief Description of the Drawings

To the accomplishment of the above and to such other objects as may hereinafter appear, the present invention is as defined in the appended claims and as described in this specification, taken together with the accompanying drawings, in which:

Figure 1 is a schematic diagram partially in perspective showing one embodiment of the invention;

Figure 2 is a schematic diagram partially in perspective of another embodiment of the invention;

Figure 3 is a schematic diagram partially in perspective of a third embodiment of the invention; and

Figure 4 is a schematic diagram partially in perspective of a fourth embodiment of the invention.

## Detailed Description of the Drawings

Figure 1 shows spectacles frame 1. It is conventional in the sense that it has temples 3, lens supports 5, and nose bridge 7 between lens supports 5.

A bar 9 extends down from frame 1. It is joined to the frame at the corner where temple 3 meets lens support 5. A mounting plate 11 is secured to bar 9. One end of plate 11 is secured to bar 9 and its other end extends laterally toward nose bridge 7. Mounted on plate 11 is an assembly 12 of two infrared sensors 13 and infrared light source 15. Light source 15 is activated by a power supply 16. The assembly 12 of light source 15 and sensors 13 is movable relative to frame 1 for a purpose to be described below. Such a device is available from the Biometrics Company of Cambridge, Massachussetts and therefore providing details herein is not deemed necessary.

As described more completely in the above-mentioned U.S. Patent No. 3,473,868, light source 15 bounces light off the sclera, iris, pupil and eyelid. To measure horizontal and vertical eye movements, sensors 13 detect the reflected light and input the resulting signals into processing circuitry 17. To this point, the description has been that of prior art apparatus.

The invention is an adaptation of the prior art apparatus by virtue of the features to be described below. Secured to one of temples 3 is an image sensitive device. This can be a CCD camera utilizing a CCD chip 19, such as the CCD222 which is a 488 x 380 element solid-state charge-coupled device area image sensor manufactured by Fairchild Camera and Instrument Corporation of Mountain View, California. CCD chip 19 may be enclosed in a suitable housing (not shown) and a lens (not shown) added to control the field of view. The remaining video circuitry for processing the signal from chip 19 is located remote from the spectacle frame. The chip is aimed frontward of the spectacle frame; that is to say, when the frame is worn, the scene detected by the chip will be that viewed by the wearer. The field of view is, of course, more limited than that of the subject. However, the items of most interest are kept by the subject generally in the central portion of his field of vision. If his peripheral vision detects something of interest, the CCD chip would be incapable of detecting it then. However, normally the subject would swivel his head promptly to get a better view thereby bringing the scene of interest within the field of view of the chip. If the arrangement is not acceptable for a particular application which may require a field of view comparable to the subject's field of vision, alternative designs can readily be developed. These include using larger chips or a plurality of chips suitably arranged. No further detailed description is believed necessary since such modifications are believed within the ability of one with ordinary skill in the art.

The signal from CCD chip 19 is input to the remaining camera electronics 21 required to convert it to a signal usable with video monitoring equipment. This involves conventional circuitry and further details are not deemed necessary.

The signal from light sensors 13 is supplied, as indicated above, to position processing circuitry 17 such as, for example, described in detail in the mentioned U.S. Patent No. 3,473,868. The processed position signal is then input to calibration circuit 25. Calibration circuit 25 includes a variable gain amplifier adjustable by the operator of the equipment. This circuit is useful to compensate for variations in eye shape of individual subjects, as described in detail below.

The signals from sensors 13 (via circuit 25) and chip 19 (via circuit 21) are input to genlock circuit 23. This circuit is a mixing circuit for superimposing the two signals. It is conventionally available off the shelf and, therefore, requires no additional details.

The signal from genlock circuit 23, which represents the superimposition of the camera signal and eye movement signal, can then be used to provide a display on monitor 27 or can be input for recordal by video tape recorder 29. Monitor 27 provides a real time display of the monitored eye movements and is also useful for calibration as described below. The taped signals provide a record for later use.

A further feature of the invention enables its use by wearers of eye glasses. More specifically, spectacle frame 1 is provided with clips 31, 32 and 33 on each lens support 5 (although shown in the drawing on only one of the lens supports for clarity of presentation). These clips are adapted to receive special corrective lenses. The lenses are inserted by hand into the clips which securely retain them in place by means of pressure. To remove the lenses, they are grasped firmly and pulled out. This arrangement may not be satisfactory for normal conditions of wear, but is adequate for the limited wear involved in connection with use of the disclosed apparatus. Other, more secure means can readily be substituted for the shown clips where the conditions of wear under test so warrant.

Various corrective lenses are kept on hand in a sufficient number of prescriptions for most eye-glass wearers. When a subject wears eye glasses, his prescription can be quickly determined by suitable equipment. The needed corrective lenses from the stock kept on hand are then inserted into clips 31, 32 and 33 for use by the subject.

It should be noted that mounted on spectacles frame 1 are only light source 15, sensors 13 and chip 19. In addition, clips 31, 32 and 33 can be used to retain corrective lenses when necessary. All the electronics for processing the eye movement signals and scene recordal signals can be packaged for carrying by the subject on his person. Alternatively it can be on a portable cart moving along with him, or it can be stationary and an electrical line used to connect the electronics with the various sensors. Preference, convenience and the specific circumstances of use dictate which alternative should be picked.

In operation, the apparatus is utilized as follows. If necessary, the appropriate corrective lensed are inserted into clips 31, 32 and 33. If the subject does not need corrective lenses, then plain glass can be inserted or the clips can be left empty. The spectacles frame is put in place on the subject's head. The subject is exposed to a visual stimulus such as a test pattern projected on a screen. His eye position is represented by a spot on the monitor generated with the components of assembly 12. Both the spot and the test pattern are displayed on monitor 27 due to their having been superimposed by genlock circuit 23. The subject is requested to look at the center of the test pattern. The position of the spot should likewise be at the center of monitor 27. If it is not, then the position of the light source and sensors assembly 12 is adjusted until the spot is positioned exactly in the center of monitor 27. Such adjustment is made by moving the light-source-and-sensors horizontally along plate 11 and/or moving plate 11 vertically along bar 9. The subject is then asked to look at any of the four corners. If the spot does not reach the corner of monitor 27, the gain of the amplifier in calibration circuit 25 is raised. If the spot overshoots, the amplifier gain is reduced until the spot is precisely where it should be.

Once calibrated, the subject is free to move around and look at anything that attracts his attention. His reactions are recorded for later analysis by VTR 29 as well as being shown in real time by monitor 27. The operator of the equipment should watch monitor 27 continuously for any sign of a problem so that an immediate repair or adjustment can be made.

Figure 2 shows a second embodiment of the invention which relies on the use of fiber optic lines. Elements in common between Figures 1 and 2 are like numbered. Thus, spectacles frame 1 is shown with the same componenets 3, 5 and 7 as well as attached components 9, 11, 15, 31, 32 and 33. A fiber optic line 39 is attached at one end to a lens 41 which is secured at the temple of spectacles frame 1. The other end of line 31 is attached to lens 43 of TV camera 45. A conventional coupling (not shown) is used between line 31 and lenses 41 and 43, respectively, and no details are therefore deemed required. Lens 41 is aimed forward of spectacles frame 1 so as to look out on the scene viewed by the wearer of the frame. This is done in the same manner as the aiming of CCD chip 19 in the Figure 1 embodiment. Thus, fiber optic line 39 conveys the image of the scene viewed by the subject to camera 45.

The subject's eye movements are monitored with lens 47 mounted on plate 11. Fiber optic line 49 is attached at one end to lens 47 and at its other end to zoom lens 51 by virtue of conventional couplings (not shown). Zoom lens 51 is coupled to focusable lens 53 of TV camera 55. The subject's eye movements are picked up by lens 47 and these are optically conveyed to camera 55. The signals from cameras 45 and 55 are combined in genlock circuit 23, in the manner above described with reference to Fig. 1, to superimpose the scene and eye movement images which are stored in VTR 29 and displayed by monitor 27.

In operation, the apparatus of Figure 2 must first be properly calibrated. This is accomplished by having the viewer peer at the center of the screen. The operator moves the light source and sensors until the spot representative of eye position is also at the center of monitor 27 (as was done for the Figure 1 embodiment). Lens 53 is turned until the dot is brought into sharp focus to compensate for subjects with unusually recessed or protruding eyes. To adjust for eye curvature the subject is requested to look at a corner of the screen, and zoom lens 51 is used to bring the dot to exactly the corresponding corner of monitor 27.

Figure 3 depicts an embodiment which permits elimination of one of the cameras shown in Figure 2. Elements in common between Figures 2 and 3 are like numbered. Thus, spectacles frame 1 has components 3, 5 and 7 and attached components 9, 11, 15, 16, 31, 32 and 33. Optical fiber line 39 and lens 41 to which it is coupled are also the same. However, the other end of line 39 is coupled to beam splitter 59 to be described below. The subject's eye movements are monitored by zoom lens 61 coupled to focusable lens 63 coupled to fiber optic line 65. The other end of line 65 is coupled to beam splitter 59. The optical output from beam splitter 59 is provided to TV camera 66 which is coupled to it.

Beam splitter 59 contains a semi-silvered mirror 67. This is a well-known device and requires no explanation. Suffice it to say that mirror 67 transmits to camera 66 light from fiber optic line 39 and reflects into camera 66 the light from fiber optic line 65. Thus, beam splitter 59 acts to optically superimpose the scene and eye movement images in camera 66. The signal from camera 66 is input to monitor 27 and VTR 29 as previously described.

Calibration of the Figure 3 embodiment is done by first centering the spot on monitor 27 in the same manner as with the previous two embodiments; that is to say, the subject is asked to look at the center of the screen and the position of the lenses 61 and 63 as well as light source 15 on plate 11 is suitably adjusted. Lens 63 adjusts for the subject's eye being recessed or bulging by focusing on the dot, as explained above with reference to lens 53

of Fig. 2. Zoom lens 61 adjusts for eye curvature by positioning the dot at the corner of monitor 27 when the subject looks at the corner of the screen.

Still another embodiment is depicted in Fig. 4. Elements in common between Fig. 1 and Fig. 4 are like numbered. Thus, spectacles frame 1 has components 3, 5 and 7 as well as clips 31, 32 and 33, and CCD chip 19. However, assembly 12 is replaced by a temple mounted assembly 71 for monitoring the subject's eye movements. Assembly 71 includes a CCD camera 73 movably mounted on vertical guide bar 74 to the frame. Focusable lens 75 is mounted on CCD camera 73, a zoom lens 77 mounted on the front of lens 75, a mirror 79 hingeably mounted at one end by bars 80 to camera 73, and light source 81 fixed to nose bridge 7 on the end of support wire 83.

CCD camera 73 includes a CCD chip, like the one used for element 19, within a suitable housing. Lenses 75 and 77 can be the same as lenses 53 and 51, respectively, in Fig. 2 and are used for the same purposes, as described below. Light source 81 bounces light off the eye and elements 77, 75 and 73 guide the reflected light into camera 73. The output of camera 73 is connected to camera circuit 83 while the signal from scene camera 19 is input to camera circuit 85. Circuits 83 and 85, like circuit 21 in the Fig,. 1 embodiment, convert the signals into a format compatible with video monitoring equipment. The outputs of circuits 83 and 85 are connected to genlock circuit 23 which feeds monitor 27 and VTR 29.

In operation, corrective lenses are placed in clips 31, 32 and 33 when required as previously described above with respect to the other embodiments. Spectacles frame 1 is then worn by the subject. The apparatus is then calibrated by asking the subject to look at the center of the scene displayed to him. If the dot representing his eye position appears anywhere other than the center of the screen of monitor 27, the vertical position of camera 73 along guide bar 74 and/or the angle of mirror 79 is (are) adjusted. When the said dot is brought to the center of the screen, lens 75 is focused to get a sharp image of the dot to correct for some irregularties of the eye, as mentioned above with regard to the Fig. 2 embodiment. The subject is then asked to look at a corner of the scene displayed to him. If the dot appears anywhere other than the corner of the screen of monitor 27, zoom lens 77 is suitably adjusted, as described above in connection with the Fig. 2 embodiment.

While a preferred embodiment of the invention is disclosed above, it is not to be treated as limiting the scope of the invention. It is apparent that several modifications can be made such as, for example, the light source and light detector assembly can be used for both eyes rather than just one. Also, various schemes can be used to adjustably mount this assembly to the spectacle frame. Furthermore, the zoom lens has been disclosed mounted on the eye-movement-monitoring devices, but can be mounted instead on the scene-monitoring devices. In addition, CCD chip 19 can be made smaller, bigger, or a combination of several can be used. Also, a signal mixing circuit other than genlock can be used to superimpose the subject's eye movements on the viewed scene. These and other modifications can be made without departing from the scope of the invention which is determined from the appended claims.

CLAIMS:

1. Apparatus for monitoring eye movements of a subject and simultaneously recording the scene being viewed by said subject comprising:

a support adapted for mounting on the head of the subject;

a light source adapted to direct light at an eye of said subject;

detector means aimed at the subject's eye for sensing reflections from the eye of light emitted by said light source;

a CCD camera secured to said support and aimed to record at least part of the scene within the subject's field of vision.

2. The apparatus of claim 1, wherein said support is a spectacle frame.

3. The apparatus of claim 1 or 2 wherein said detector means comprises two detectors and further comprising a position-processing-circuit means coupled to said detectors for converting the output of said detectors to a signal for use on video equipment to display eye position.

4. The apparatus of any of claims 1 to 3, wherein said detector means comprises a further CCD camera.

5. The apparatus of claim 4, further comprising a focus lens and a zoom lens affixed to the further CCD camera.

6. The apparatus of claim 4, 5 or 6, wherein the further CCD camera is secured to the temple portion of the spectacle frame and said light source is secured to the nose bridge of said spectacle frame and further comprising a mirror secured to the spectacle frame for directing reflected light from the eye into said further CCD camera.

7. Apparatus for monitoring eye movements of a subject and simultaneously recording the scene being viewed by said subject comprising:

a frame adapted for mounting on the head of the subject of a type normally used to support corrective lenses and having a pair of temples, two lens supports with each attached at one end to a temple and at the other to a nose bridge;

a light source secured to said frame and adapted to direct light at an eye of said subject when the frame is worn in place by the subject;

detector means secured to said frame for receiving light reflected by the subject's eye when the frame is worn in place by the subject for sensing reflections from the eye of light emitted by said light source; and

means secured to said frame and aimed to record at least part of the scene within the subject's field of vision.

8. Apparatus for monitoring eye movements of a subject and simultaneously recording the scene being viewed by said subject comprising:

a support adapted for mounting on the head of said subject;

a light source secured to said support and adapted to direct light at an eye of said subject when the support is worn on the subject's head;

detector means secured to said support and aimed at the subject's eye when worn on the head;

means secured to said frame and aimed to record at least part of the scene within the subject's field of vision; and

means on said support for securing corrective lenses to said frame and facilitating the installation and removal of such lenses.

9. Apparatus for monitoring eye movements of a subject and simultaneously recording the scene being viewed by said subject comprising:

(a) a spectacle frame having a pair of temples, a pair of lens supports and a nose bridge;

(b) a light source adjustably secured to said frame and aimed at the eye of said subject when the frame is worn;

(c) detector means adjustably secured to said frame for receiving light from the subject's eye when said frame is worn;

(d) a CCD camera secured to said frame and aimed to record at least part of the scene within the subject's field of vision;

(e) camera circuitry coupled to said CCD camera for converting its signal to one compatible with video equipment for display;

(f) detector circuitry coupled to said detector means for providing an eye movement signal compatible with video equipment for display; and

(g) means coupled to the detector circuitry and camera circuitry for combining the same signal and eye movement signals in a superimposed manner.

10. Apparatus for monitoring eye movements of a subject and simultaneously recording the scene being viewed by said subject comprising:

(a) a spectacle frame having a pair of temples, a pair of lens supports and a nose bridge;

(b) a light source adjustably secured to said frame and aimed at the eye of said subject when the frame is worn;

(c) detector means adjustably secured to said frame for receiving light from the subject's eye when said frame is worn;

(d) a first optical lens secured to said frame and aimed to record at least a part of the scene within the subject's field of vision;

(e) means for conveying light from said lens;

(f) means for combining the subject's eye movements from the detector means with light from said conveying means to provide a signal compatible with video equipment for display; and

(g) means for displaying the combined scene and subject eye movements in superimposed fashion.

FIG. 1

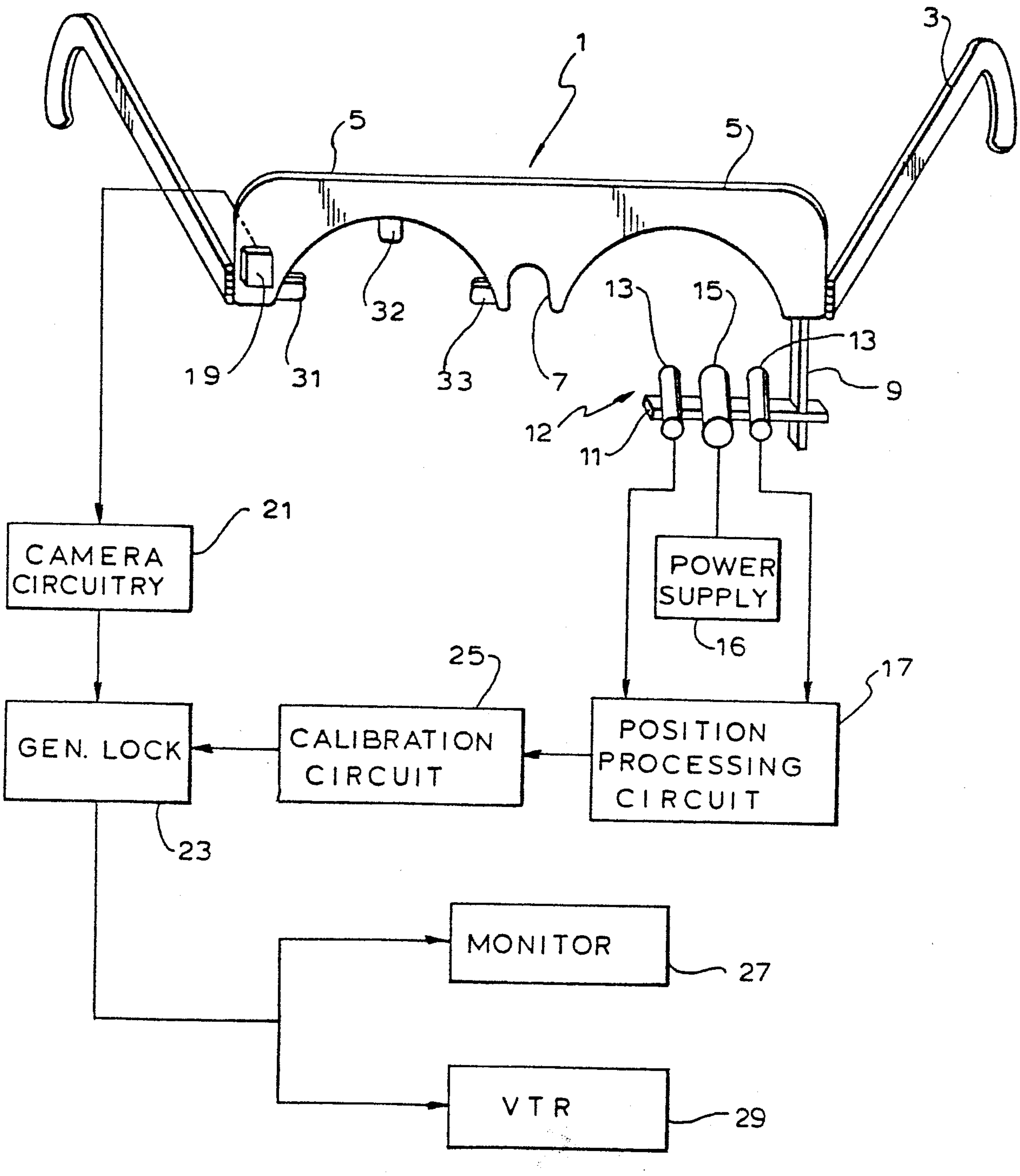
1
3
5
5
7
9
11
12
13
13
15
16
17
19
21
23
25
27
29
31
32
33
CAMERA CIRCUITRY
POWER SUPPLY
GEN. LOCK
CALIBRATION CIRCUIT
POSITION PROCESSING CIRCUIT
MONITOR
VTR

FIG. 2

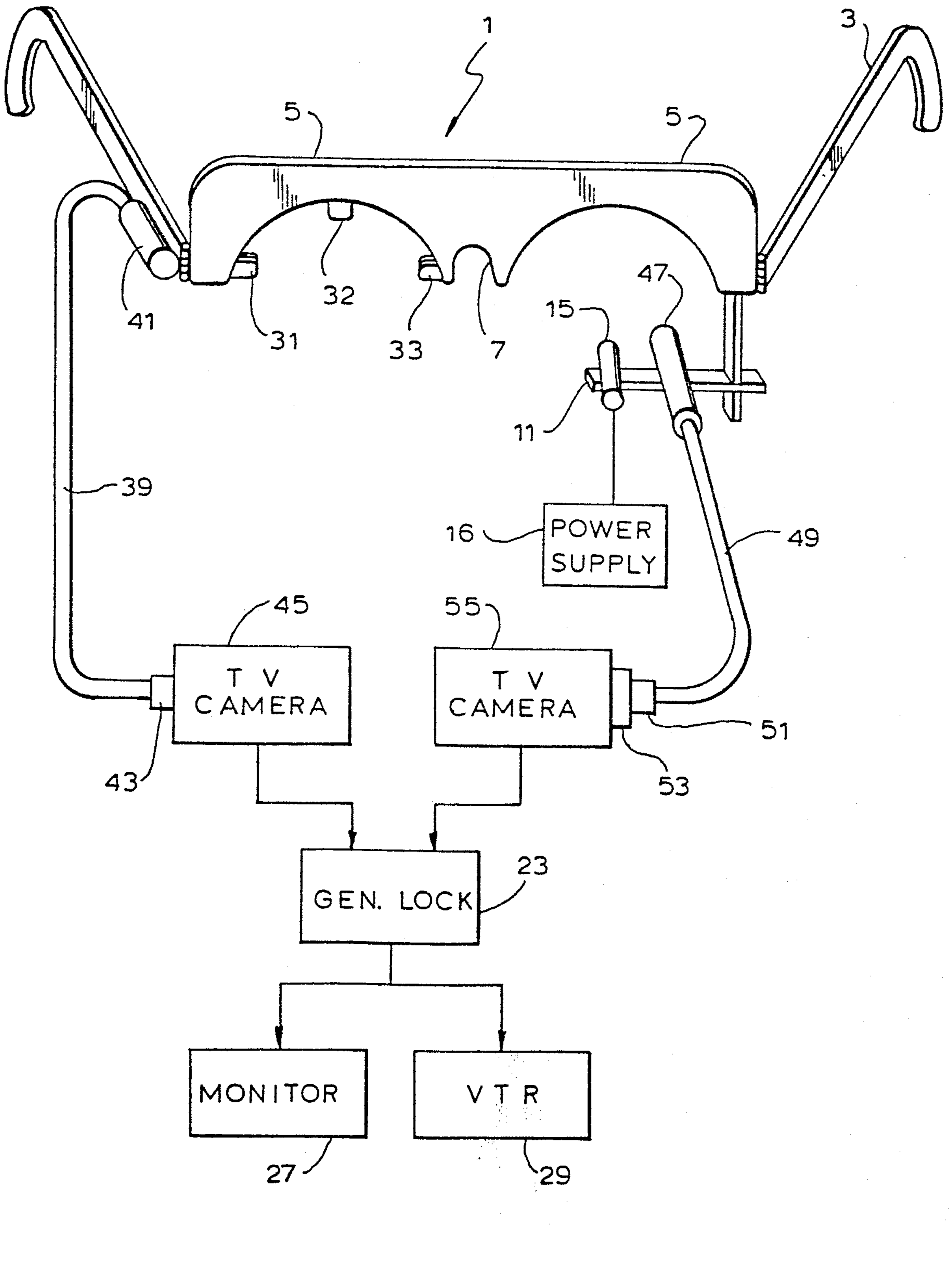
1
3
5
5
41
32
31
33
7
47
15
11
39
16
POWER SUPPLY
49
45
55
T V CAMERA
T V CAMERA
51
43
53
23
GEN. LOCK
MONITOR
VTR
27
29

FIG.3

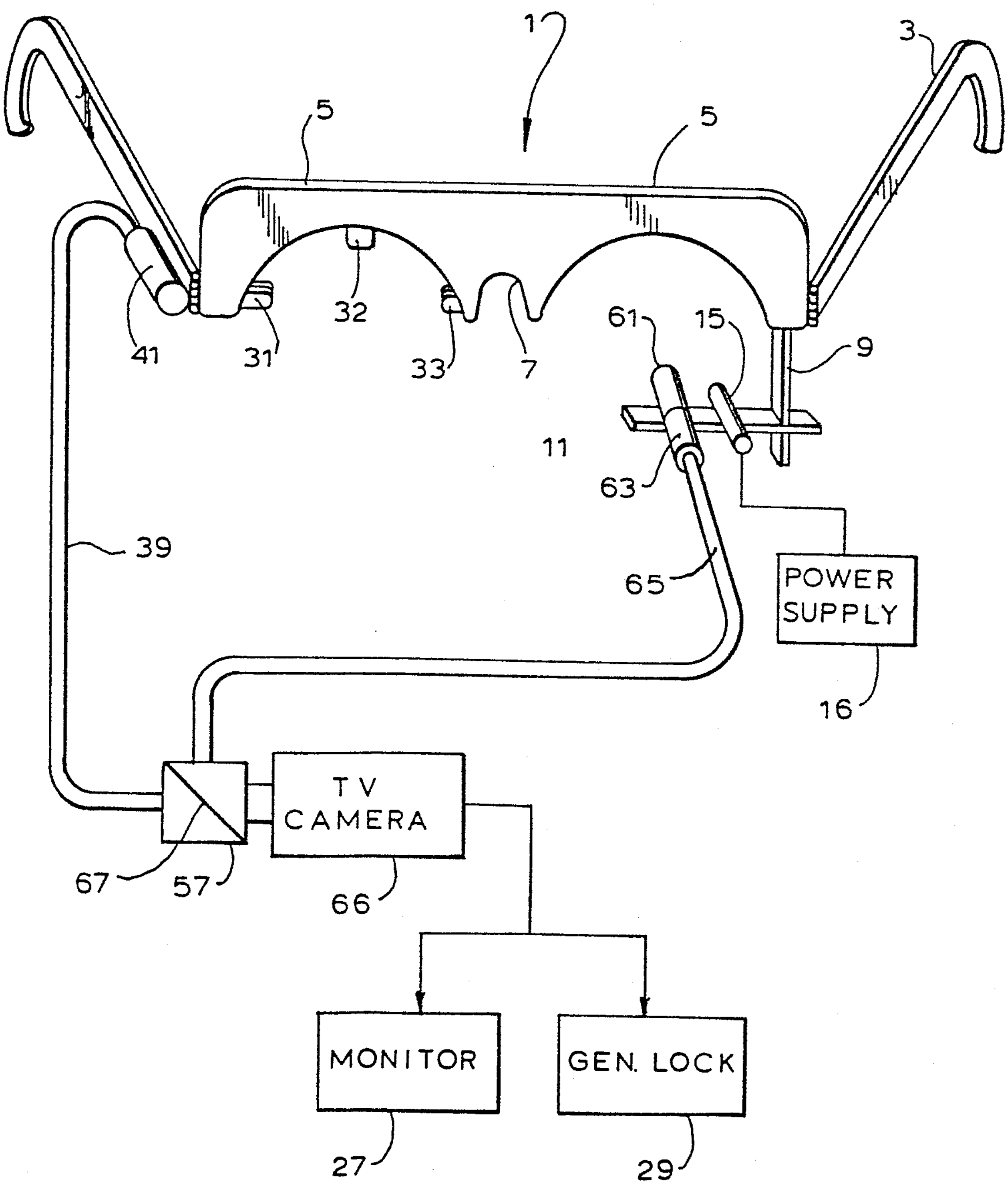
1
3
5
5
41
31
32
33
7
61
15
9
11
63
39
65
POWER SUPPLY
16
TV CAMERA
67
57
66
MONITOR
GEN. LOCK
27
29

F I G. 4

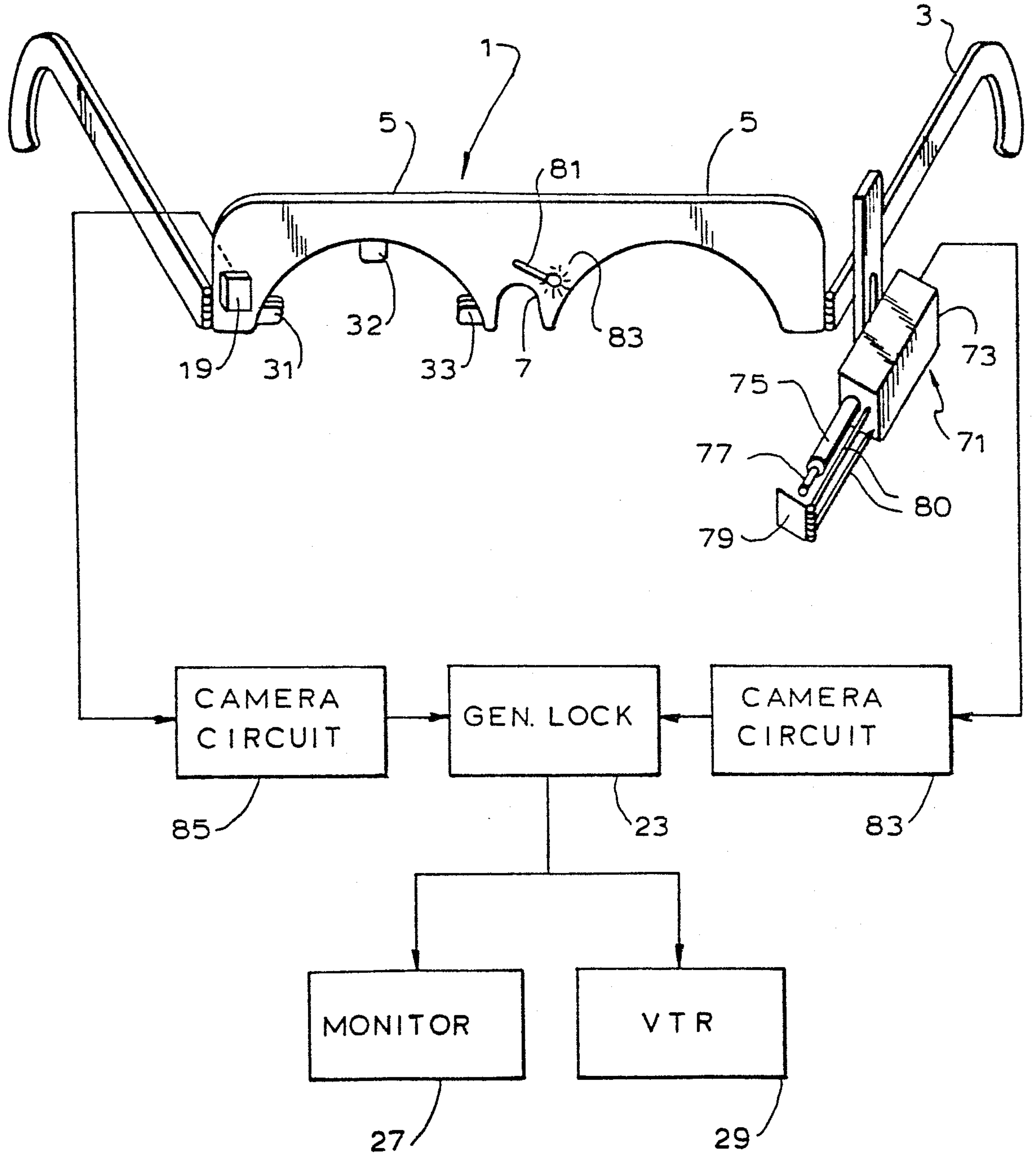
1
3
5
5
81
32
83
19
31
33
7
73
75
71
77
80
79
CAMERA CIRCUIT
GEN. LOCK
CAMERA CIRCUIT
85
23
83
MONITOR
VTR
27
29